# EUROPEAN PATENT SPECIFICATION

(11) **EP 3 034 085 B1**
(45) Date of publication and mention of the grant of the patent: **23.10.2019**
(21) Application number: 14836115.7
(22) Date of filing: 07.08.2014
(51) Int. Cl.: A61K 31/737, A61K 31/722, A61K 31/727, A61K 31/728, A61L 15/16, A61P 17/02, A61P 7/04

(54) **DRUG CONTAINING CATIONIZED CHITOSAN**
ARZNEIMITTEL MIT KATIONISIERTEM CHITOSAN
MÉDICAMENT CONTENANT DU CHITOSANE CATIONISÉ

(30) Priority: 13.08.2013 JP 2013168324
(43) Date of publication of application: 22.06.2016
(73) Proprietor: Seikagaku Corporation, Tokyo 100-0005 (JP)
(72) Inventor: OTA, Naoki, Tokyo 100-0005 (JP); MUNEMURA, Yuichi, Tokyo 100-0005 (JP); OTSUKA, Akira, Tokyo 100-0005 (JP)
(74) Representative: Müller-Boré & Partner Patentanwälte PartG mbB
(86) International application number: PCT/JP2014/070946
(87) International publication number: WO 2015/022907

(56) References cited:
- WO-A1-2012/171125
- WO-A1-2012/171125
- JP-A- 2004 231 578
- JP-A- 2012 529 506
- KAMIL KAMINSKI ET AL: "Chitosan Derivatives as Novel Potential Heparin Reversal Agents", JOURNAL OF MEDICINAL CHEMISTRY, vol. 53, no. 10, 27 May 2010 (2010-05-27), pages 4141-4147, XP055342887, US ISSN: 0022-2623, DOI: 10.1021/jm1001666
- JORGE ALMODOVAR ET AL: "Layer-by-Layer Assembly of Polysaccharide-Based Polyelectrolyte Multilayers: A Spectroscopic Study of Hydrophilicity, Composition, and Ion Pairing", BIOMACROMOLECULES, vol. 12, no. 7, 11 July 2011 (2011-07-11), pages 2755-2765, XP055342680, US ISSN: 1525-7797, DOI: 10.1021/bm200519y
- S. HEIN ET AL: "Chitosan composites for biomedical applications: status, challenges and perspectives", MATERIALS SCIENCE AND TECHNOLOGY, vol. 24, no. 9, 1 September 2008 (2008-09-01), pages 1053-1061, XP055315554, GB ISSN: 0267-0836, DOI: 10.1179/174328408X341744
- KWEON D-K ET AL: "Preparation of water-soluble chitosan/heparin complex and its application as wound healing accelerator", BIOMATERIALS, ELSEVIER SCIENCE PUBLISHERS BV., BARKING, GB, vol. 24, no. 9, 1 April 2003 (2003-04-01), pages 1595-1601, XP004404215, ISSN: 0142-9612, DOI: 10.1016/S0142-9612(02)00566-5
- DENUZIERE A ET AL: "Interactions between chitosan and glycosaminoglycans (chondroitin sulfate and hyaluronic acid): Physicochemical and biological studies", ANNALES PHARMACEUTIQUES FRAN CR CAISES, ELSEVIER MASSON, FR, vol. 58, no. 1, 1 January 2000 (2000-01-01), pages 47-53, XP009097772, ISSN: 0003-4509
- KAMINSKI K ET AL.: 'Chitosan derivatives as novel potential heparin reversal agents.' JOURNAL OF MEDICINAL CHEMISTRY vol. 53, 2010, pages 4141 - 4147, XP008173387
- HEVELINE D ET AL.: 'Antiadhesive and antibacterial multilayer films via layer-by- layer assembly of TMC/heparin complexes.' BIOMACROMOLECULES vol. 13, no. 11, 2012, pages 3711 - 3722, XP055315553
- HEIN S: 'Chitosan composites for biomedical applications: status, challenges and perspectives' MATERIALS SCIENCE AND TECHNOLOGY vol. 24, no. 9, 2008, pages 1053 - 1061, XP055315554
- KWEON DK ET AL.: 'Preparation of water-soluble chitosan/heparin complex and its application as wound healing accelerator.' BIOMATERIALS vol. 24, 2003, pages 1595 - 1601, XP004404215

## Description

### Technical Field

The present invention relates to a novel application of cationized chitosan.

### Background Art

PTL 1 describes a skin wound healing agent in which chitosan is combined with a polysaccharide such as heparin or heparan sulfate and indicates that the polysaccharide in this healing agent is immobilized to the chitosan by ionic bonding and that this healing may be presented in the form of a powder, ointment, paste, gel, suspension, solution, or film. However, PTL 1 neither describes nor suggests the use of a "cationized chitosan", which is provided by the introduction of a cationic group into chitosan, or that cationized chitosan inhibits the hemorrhagic activity that originates from sulfated glycosaminoglycans. NPL1 describes ionic complexes of chitosan and sulfated glycosaminoglycans such as chondroitin sulfate for improving the wound-healing acceleration and for skin and cartilage recovery. However, NPL1 does not suggest to use cationized chitosans.

It is known, on the other hand, that cationized chitosan is used in, for example, a hair cosmetic (PTL 2), a wood preservative (PTL 3), a primer for improving the corrosion resistance of metals (PTL 4), a packaging material for electronic component cases (PTL 5), and an antimicrobial fiber (PTL 6). Its use for an immunoadjuvant is also known (PTL 7). However, none of PTL 2, PTL 3, PTL 4, PTL 5, PTL 6, and PTL 7 indicates or suggests that cationized chitosan inhibits the hemorrhagic activity originating from sulfated glycosaminoglycans or the use of a complex of cationized chitosan and a sulfated glycosaminoglycan as an active ingredient in a drug for the treatment of biological tissues.

In addition, use for wound healing is described in PTL 8, PTL 9, and PTL 10 for chondroitin sulfate B (dermatan sulfate), chondroitin sulfate E, and low molecular weight keratan sulfate, respectively. However, none of PTL 8, PTL 9, and PTL 10 describes or suggests the use of cationized chitosan or the use of a complex of cationized chitosan and these sulfated glycosaminoglycans as an active ingredient in a drug for the treatment of biological tissues. Moreover, as shown in the reference examples in this Description, a promoting effect on wound healing was not observed for cationized chitosan by itself.

### Citation List

### Non-Patent Literature

[NPL1] DENUZIERE A. et al, ANNALES PHARMACEUTIQUES FRANCAISES, vol. 58, no. 1, 2000, pages 47-53

### Patent Literature

[PTL1] Japanese Translation of PCT Application No. H10-502665
[PTL2] Japanese Patent No. 4,632,040
[PTL3] Japanese Patent No. 2,564,466
[PTL4] Japanese Patent No. 4,081,276
[PTL5] Japanese Patent No. 5,010,097
[PTL6] Japanese Patent Application Laid-open No. 2000-219605
[PTL7] WO 2009/013972
[PTL8] Japanese Patent Application Laid-open No. 2001-187740
[PTL9] Japanese Patent Application Laid-open No. 2004-18390
[PTL10] WO 96/016973

### Summary of Invention

Evaluations were not performed in vivo in PTL 1, and when the present inventors carried out additional testing in vivo, it was found, as described below, that substantial hemorrhaging occurred when this agent was applied at the wound site and it was thus not suitable for practical use.

A task for the present invention is to provide an art that inhibits the hemorrhagic activity exhibited by sulfated glycosaminoglycans, as well as a material, a medical material, and a drug that use a sulfated glycosaminoglycan while being free of the hemorrhaging problems caused by sulfated glycosaminoglycans and that can exhibit an excellent therapeutic effect on biological tissues.

Upon carrying out intensive investigations directed to solving the aforementioned task, the present inventors discovered that cationized chitosan inhibits the hemorrhagic activity originating from sulfated glycosaminoglycans and that, when a complex of a cationized chitosan and a sulfated glycosaminoglycan is prepared and used with an organism, the hemorrhagic activity is remarkably inhibited and an excellent therapeutic effect on biological tissues is exhibited. The present invention was achieved based on this discovery.

That is, the aforementioned task is solved by the present invention, which is illustrated in the following.

In a first embodiment present invention relates to a complex comprising N-(2-hydroxypropyl)-3-trimethylammonium chitosan chloride and chondroitin sulfate A, chondroitin sulfate B or chondroitin sulfate E.

In a second embodiment present invention relates to a wound dressing that comprises the complex as defined above.

In a third embodiment present invention relates to a drug for use in healing wound, the drug comprising the complex as defined above as an active ingredient.

The inhibitor of the present invention is very useful because the inhibitor can remarkably inhibit hemorrhagic activity that originates from sulfated glycosaminoglycans while retaining the therapeutic effect for biological tissues.

The dressing of the present invention is very useful because it has excellent wound dressing properties, is also highly biocompatible, and can be conveniently and hygienically removed. The dressing of the present invention and the drug of the present invention are both very useful because they substantially inhibit the hemorrhaging that originates from sulfated glycosaminoglycans while exhibiting an excellent therapeutic effect on biological tissues.

The complex of the present invention is very useful because it can provide a source material for the dressing of the present invention and the drug of the present invention.

The following abbreviations are used in this Description.
CT⁺ : cationized chitosan
Hep/CT : complex of chitosan and heparin
Hep/CT⁺ : complex of cationized chitosan and heparin
CS-E/CT : complex of chitosan and chondroitin sulfate E
CS-E/CT⁺ : complex of cationized chitosan and chondroitin sulfate E
CS-A/CT⁺ : complex of cationized chitosan and chondroitin sulfate A
CS-B/CT⁺ : complex of cationized chitosan and chondroitin sulfate B
CS-C/CT⁺ : complex of cationized chitosan and chondroitin sulfate C
CS-D/CT⁺ : complex of cationized chitosan and chondroitin sulfate D
KPS/CT⁺ : complex of cationized chitosan and keratan polysulfate

### Brief Description of Drawings

Fig. 1 shows the status of hemorrhaging in the use of complexes of chitosan with different sulfated glycosaminoglycans and the use of complexes of cationized chitosan with different sulfated glycosaminoglycans (photograph).
Fig. 2 shows an evaluation of hemorrhaging in the use of complexes of chitosan with different sulfated glycosaminoglycans and the use of mixtures (complexes) of cationized chitosan with different sulfated glycosaminoglycans; the hemorrhaging and mortality due to blood loss were reduced by changing the Hep/CT to Hep/CT⁺; and, hemorrhaging was completely inhibited by changing the CSE/CT to CSE/CT⁺.
Fig. 3 shows the biological tissue repair effect for complexes of cationized chitosan with different sulfated glycosaminoglycans; a promotion of healing at the treated wound was observed with Hep/CT⁺, CSE/CT⁺, and CSE/CT; and the evaluation could not be performed with Hep/CT due to animal mortality.
Fig. 4 shows the biological tissue repair effect for complexes of cationized chitosan with different sulfated glycosaminoglycans; and significant hemorrhaging was absent and a promotion of wound healing was observed with Hep/CT⁺, CS-A/CT⁺, CS-B/CT⁺, CS-C/CT⁺, CS-D/CT⁺, CS-E/CT⁺, and KPS/CT⁺.
Fig. 5 shows the wound healing effect of cationized chitosan; and a promotion of wound healing was not obtained for CT⁺ alone, and healing was thus delayed (hemorrhaging from the wound site was not observed).

### Description of Embodiments

### [1] The inhibitor of the present invention

The inhibitor of the present invention is an inhibitor of hemorrhagic activity that originates from a sulfated glycosaminoglycan, and has a cationized chitosan as an active ingredient.

The cationized chitosan that is an active ingredient of the inhibitor of the present invention has a positive charge in the chitosan molecule. For example, it may be provided by the introduction of a positive charge-bearing functional group into chitosan so as to impart a positive charge thereto. The introduction of a positive charge-bearing functional group can be carried out by the introduction of a quaternary ammonium group at the amino group or hydroxyl group in the chitosan molecule or by the replacement of the hydrogen atom on the amino group in the chitosan molecule with the hydrocarbon group so as to obtain a quaternary ammonium group. When introduction is through covalent bonding, there are no particular limitations on this method other than that a covalent bond is formed by a chemical reaction.

An inhibitory effect on the hemorrhagic activity originating from the sulfated glycosaminoglycan can be confirmed by the method described in the examples in this Description.

A chitosan bearing a permanent positive charge can be obtained by the introduction of a quaternary ammonium group (quaternary chitosan). Here, "bearing a permanent positive charge" means that a positive charge can be present without an effect, for example, from the pH of the surroundings. The quaternary chitosan according to the present invention is N-(2-hydroxypropyl)-3-trimethylammonium chitosan chloride and further disclosed is (N,N,N)-trimethylchitosan chloride.

This cationized chitosan can be a commercially available cationized chitosan used as such or can be prepared, using chitosan as a starting material, by methods that are themselves known. For example, the N-(2-hydroxypropyl)-3-trimethylammonium chitosan chloride according to the invention can be produced by the methods described in, for example, Biomaterials 24, 2003, 5015; Carbohydrate Research 339, 2004, 313; Coloration Technology 120, 2004, 108; Colloids and Surfaces A: Physicochemical Engineering Aspects 242, 2004, 1; Polymer Journal 32, 2000, 334; and International Journal of Biological Macromolecules 34, 2004, 121-126. The (N,N,N)-trimethylchitosan chloride can be produced by the methods described in, for example, Carbohydrate Polymers 5, 1985, 297; International Journal of Biological Macromolecules 8, 1986, 105; Carbohydrate Polymers 24, 1994, 209; Carbohydrate Polymers 36, 1998, 157; and Drug Development and Industrial Pharmacy 27, 2001, 373.

With regard to the production of the cationized chitosan from chitosan, there is also no particular limitation on the apparent viscosity of the chitosan that can be used as a starting material. For example, for the 0.5 w/v% solution using a 0.5% aqueous acetic acid solution as the solvent, this can be, for example, not more than 2,000 mPa · s and preferably not more than 1,000 mPa · s.

The inhibitor of the present invention can be produced from the cationized chitosan as such or with the optional blending therewith as appropriate of other components that will not impair the effects of the present invention. The dosage form of the inhibitor of the present invention is also not particularly limited, and a desired form can be used.

The thusly produced inhibitor of the present invention is used to inhibit hemorrhagic activity that originates from sulfated glycosaminoglycans.

The sulfated glycosaminoglycan whose hemorrhagic activity is inhibited by the inhibitor of the present invention is chondroitin sulfate A, chondroitin sulfate B and chondroitin sulfate E. Also disclosed are heparin, heparan sulfate, keratan sulfate, chondroitin sulfate, sulfated hyaluronic acid, keratan polysulfate, chondroitin sulfate C, chondroitin sulfate D, and oversulfated chondroitin sulfate. Chondroitin sulfate E is preferred among the sulfated glycosaminoglycans according to the invention.

There are also no particular limitations on the weight-average molecular weight of the sulfated glycosaminoglycans, and this can be, for example, generally from about 500 to 10,000,000 and preferably from about 1,000 to 8,000,000.

These sulfated glycosaminoglycans may be natural or synthetic. These sulfated glycosaminoglycans are all commercially available and their production methods are also known and they may thus be easily acquired.

In order to inhibit the hemorrhagic activity of a sulfated glycosaminoglycan, it is sufficient to bring the inhibitor of the present invention into contact with the sulfated glycosaminoglycan. There are no particular limitations on this contact as long as it is a state in which the cationized chitosan molecule in the inhibitor of the present invention comes into contact with the sulfated glycosaminoglycan molecule. In an example of such a state, the respective solutions are prepared and the two are mixed. Or, this may be a state in which the cationized chitosan is applied to a biological tissue that requires treatment and where sulfated glycosaminoglycan is present. For example, preferably the formation of ionic bonds between the cationized chitosan molecule and the sulfated glycosaminoglycan molecule is brought about by an adequate execution of such contact. Among the preceding, the formation of a polyionic complex of the two molecules is preferred.

The inhibitor of the present invention can inhibit the hemorrhagic activity exhibited by sulfated glycosaminoglycans while keeping the wound healing activity of sulfated glycosaminoglycans intact.

### [2] The complex of the present invention

The complex of the present invention is a complex comprising a cationized chitosan and a sulfated glycosaminoglycan according to claim 1.

The cationized chitosan and the sulfated glycosaminoglycan constituting this complex are the same as described above in "The inhibitor of the present invention".

This complex can be produced by bringing a cationized chitosan into contact with a sulfated glycosaminoglycan. There are no particular limitations on this contact as long as it is a state in which the cationized chitosan molecule comes into contact with the sulfated glycosaminoglycan molecule and forms a complex. For example, the respective solutions, preferably aqueous solutions, may be prepared and these two may be mixed to directly form the complex, or the two may be brought into contact in the presence of a salt followed by removal of the salt by dialysis or another method to form a complex of the two. The mixing method, conditions, and so forth may be adjusted as appropriate within a range in which a complex comprising the cationized chitosan and sulfated glycosaminoglycan can be formed. For specifics, reference can be made to the method described in the examples of this Description. With such a method, ionic bonds can be formed between the two molecules and a polyionic complex can be formed. After its production as described in the preceding, the complex of the present invention may be isolated, dried, purified, and so forth.

The compositional ratio between the cationized chitosan and the sulfated glycosaminoglycan in this complex is also not limited and can be exemplified by approximately 1 : 0.001 to 1 : 1000 (molar ratio).

The thusly produced complex of the present invention can be used as a constituent material for the dressing of the present invention and the drug of the present invention, which are described below.

### [3] The dressing of the present invention

The dressing of the present invention is a wound dressing that contains the complex of the present invention. The molecules (cationized chitosan, sulfated glycosaminoglycan) constituting the complex of the present invention that is contained in the dressing of the present invention, the preferred state of bonding in the complex between the cationized chitosan and the sulfated glycosaminoglycan molecule, and so forth, are as described above in "The complex of the present invention". The dressing of the present invention may be made of this complex of the present invention as such or as necessary may be provided by the incorporation thereto as appropriate of other components that do not impair the effects of the present invention, for example, a pharmaceutically acceptable carrier and so forth.

The form of the dressing of the present invention is not limited as long as it is capable of covering the wound site, and it can be produced by a known method in correspondence to the desired form. For example, when the complex of the present invention is provided in the form of a solution, suspension, or gel, the dressing of the present invention may then take the form of the solution, suspension, or gel as such. In addition, the dressing of the present invention may be provided in the form of the dried material obtained, for example, by drying, using a method such as lyophilization, the complex of the present invention provided in solution, suspension, or gel form. The dressing of the present invention may be provided by combining the complex of the present invention with a common wound dressing. Known methods may also be used for processing, molding, and so forth.

Other forms for the dressing of the present invention can be exemplified by powders, granules, sheets, sponges, meshes, and so forth. Additional examples are forms such as ointments, pastes, and gels.

When a wound site is covered with the thusly prepared dressing of the present invention, wound healing can be promoted while hemorrhaging from the wound site is inhibited because the wound healing activity of the sulfated glycosaminoglycan is preserved intact while the hemorrhagic activity of the sulfated glycosaminoglycan is inhibited.

### [4] The drug of the present invention

The drug of the present invention is a drug for the treatment of a biological tissue and has the complex of the present invention as an active ingredient. The molecules (cationized chitosan, sulfated glycosaminoglycan) constituting the complex of the present invention, which is an active ingredient in the drug of the present invention, the preferred state of bonding between the cationized chitosan and the sulfated glycosaminoglycan in the complex, and so forth, are as described above in "The complex of the present invention". The drug of the present invention may be made of this complex of the present invention as such or as necessary may be provided by the incorporation thereto as appropriate of other components that do not impair the effects of the present invention.

The target for the application of the drug of the present invention is an animal for which treatment of a biological tissue is required, but is not otherwise particularly limited; mammals are preferred and humans are preferred thereamong.

The biological tissue to which the drug of the present invention is applied is a biological tissue requiring, for example, treatment, tissue repair, and so forth, but is not otherwise particularly limited and can be exemplified by the skin, organs, bone, and so forth. Among these, application to the skin and organs, which are highly vascularized biological tissues and present a high risk of hemorrhage, is preferred and application to the skin is preferred. The condition of the biological tissue requiring treatment, tissue repair, and so forth is also not particularly limited and can be exemplified by wounds, ulcers, and so forth. Application to wounds is preferred among the preceding. That is, the drug of the present invention is preferably a wound healing agent and is more preferably a wound healing agent for skin.

In the case of application to wounds, the cause of the injury is not particularly limited, and the drug of the present invention may be used for the treatment of acute wounds as well as chronic wounds. The type of wound can be exemplified by cuts, lacerations, chop wounds, abrasions, crush wounds, contusions, bruises, gunshot wounds, explosive injuries, puncture wounds, impalement wounds, bite wounds, burns, frostbite, chemical burns, surgical wounds, pressure sores, ulcers, and spontaneously occurring wounds.

The method of using the drug of the present invention is a modality whereby the drug of the present invention exhibits a therapeutic effect at the site in a biological tissue that requires, e.g., treatment, tissue repair, and so forth, but is not otherwise particularly limited. An example is the method of direct application to the site (for example, the wound site in the case of a wound). This method of application can be exemplified by covering the site with the drug of the present invention. The covering technique can be exemplified by pasting, coating, spraying, and other techniques.

The dosage form of the drug of the present invention can be selected by the individual skilled in the art from among the various known dosage forms as appropriate in conformity to the method of using the drug of the present invention. For example, just as for the previously described dressing of the present invention, the drug of the present invention may take the form of a solution or suspension of the complex of the present invention, which is an active ingredient in the drug of the present invention, or the drug of the present invention may be provided by suitably molding a dry material form obtained by drying the preceding by a method such as lyophilization. The dressing of the present invention may also be used as such as the drug of the present invention.

The drug of the present invention may be applied only a single time to the biological tissue, and after application it may as necessary be, for example, supplemented, exchanged, removed, and so forth. It may also be applied continuously.

### Examples

The present invention is specifically described in the following using examples. However, the technical scope of the present invention is not limited to or by this.

### < Example 1 > Production of cationized chitosan

### (1) The chitosan

A commercially available chitosan (Wako Pure Chemical Industries, Ltd., CT300, flake, apparent viscosity at a 0.5 w/v% concentration using a 0.5 % aqueous acetic acid solution as solvent = 257 mPa · s) was used for the chitosan.

### (2) Production of microparticulated chitosan

A microparticulated chitosan was produced according to the method described in Japanese Patent Application Laid-open No. H9-143203 for use as a starting material for producing cationized chitosan. Specifically, 10.0 g of the aforementioned chitosan was added to 600 mL of deionized water and 10 mL of acetic acid was then added. After bringing about dissolution by stirring at room temperature, the resulting solution was filtered using a glass filter. 20.0 g of ammonium sulfate was added to the filtrate and microparticles of chitosan were precipitated by stirring and mixing.

Once the precipitation was completed, 5 M NaOH was added until the pH reached about 8 to 9 in order to neutralize the acetic acid. After this, the precipitated microparticulated chitosan was separated from the solvent using a glass filter. The separated microparticulated chitosan was washed with hydrous ethanol to remove the salts contained therein. After this washing had been repeated several times, the moisture entrained in the microparticulated chitosan was removed by washing with acetone. This was followed by drying at 40°C under reduced pressure to obtain 9.8 g of microparticulated chitosan (as a powder).

### (3) Production of cationized chitosan

Cationized chitosan was produced according to the method described in PTL 3. Specifically, 150 mL of 80 v/v% isopropyl alcohol was added to 9.0 g of the microparticulated chitosan obtained in (2) above and stirring was carried out at 60°C. After this, 9.0 mL of glycidyltrimethylammonium chloride (SY-GTA80 from Sakamoto Yakuhin Kogyo Co., Ltd.) was added and a reaction was run by stirring for 7 hours at 60°C. After the completion of the reaction, the reaction product was separated from the reaction solvent using a glass filter. The separated reaction product was washed with 20 v/w ethanol and acetone. This was followed by drying the reaction product at 40°C under reduced pressure to obtain 14.2 g of a cationized chitosan (N-(2-hydroxypropyl)-3-trimethylammonium chitosan chloride, which is a quaternary chitosan).

### < Example 2 > Production of complexes comprising cationized chitosan and sulfated glycosaminoglycan

### (1) Production of a complex of cationized chitosan and heparin (reference example)

A complex (Hep/CT⁺) of cationized chitosan and heparin (Wako Pure Chemical Industries, Ltd., weight-average molecular weight of approximately 10,000), which is a type of sulfated glycosaminoglycan, was prepared. Specifically, 220 mg of the cationized chitosan produced in (3) in Example 1 and 150 mg of the heparin were simultaneously dissolved in 8 mL of a 5.0 w/v% sodium chloride solution and thorough mixing was carried out until the two components reached to homogeneous condition. The resulting mixture was poured into a mold that had been surface-treated with Teflon (registered trademark) and was held at quiescence until the mixture completely reached to smoothing condition. This mixture was subsequently introduced along with the mold into a large amount of deionized water and a desalting dialysis (about 20 hours) was carried out from the contact surface between the mixture and the deionized water to form a complex of the cationized chitosan and heparin (polyionic complex in which the cationized chitosan molecule and heparin molecule were ionically bonded) and obtain a molded gel. This gel was white in each instance. This gel was also lyophilized to obtain a spongy dry material. This dry material was white in each instance. The weight of the obtained dry material was approximately 270 mg.

### (2) Production of complexes of cationized chitosan with different sulfated glycosaminoglycans

Complexes (polyionic complexes in which the two molecules were ionically bonded) of cationized chitosan and sulfated glycosaminoglycan were prepared and the spongy dry materials were obtained proceeding as in (1) above, but using different sulfated glycosaminoglycans (chondroitin sulfate A, chondroitin sulfate B (dermatan sulfate), chondroitin sulfate C, chondroitin sulfate D, chondroitin sulfate E, and keratan polysulfate, all from Seikagaku Corporation, with weight-average molecular weights of, respectively, approximately 10,000, approximately 30,000, approximately 40,000, approximately 30,000, approximately 70,000, and approximately 10,000) in place of the heparin used in (1) above. The weight of the obtained dry material was in each case approximately 310 mg for the complex with chondroitin sulfate A (CS-A/CT⁺), the complex with chondroitin sulfate B(CS-B/CT⁺), the complex with chondroitin sulfate C(CS-C/CT⁺), the complex with chondroitin sulfate E(CS-E/CT⁺), and the complex with keratan polysulfate (KPS/CT⁺), and was approximately 300 mg for the complex with chondroitin sulfate D(CS-D/CT⁺). The complexes CS-A/CT+, CS-B/CT+ and CS-E/CT+ are invention examples, the other complexes are reference examples.

### < Example 3 > Production of wound dressings and drugs for treating biological tissues

The dry materials of the cationized chitosan and sulfated glycosaminoglycan complexes prepared in Example 2 were cut to approximately 1 cm² and then used in the Example 4 described below.

### < Reference Example 1 > Production of control substances

### (1) Production of spongy chitosan

A spongy chitosan was obtained as follows based on the method described in PTL 1.

A 2 w/v% chitosan solution was prepared by adding the chitosan to a 2.0 v/v% aqueous acetic acid solution under sufficient stirring condition.

10.00 g of this chitosan solution (contained 200 mg chitosan) was cast out onto a polypropylene petri dish (diameter: 85 mm) and the chitosan solution was held at quiescence until it completely reached to smoothing condition. The chitosan solution was then frozen; 2 M NaOH (10 mL) was added to the frozen chitosan solution; and allowed to stand for approximately 3 hours at room temperature. After confirming that the solution had changed to white gel condition, the gel was peeled from the bottom of the petri dish and the entire gel was immersed in a 2 M NaOH solution and allowed to stand for 4 days at room temperature. The surface of this white gel was then washed with deionized water followed by immersion of the gel in a large amount of deionized water and stirring the deionized water with a magnetic stirrer (600 rpm) to remove the excess NaOH. This processes were repeated several times until the pH of the deionized water had stabilized near neutrality (final pH = 6.5). The white gel was then lyophilized to obtain a spongy chitosan. Its weight was 180 mg.

### (2) Production of the complex of chitosan and sulfated glycosaminoglycan (reference examples)

A heparin solution (pH 6.6) was prepared by dissolving 1067 mg heparin in 220 mL of a 0.2 M phosphate buffer solution (pH 6.4).

20 mL of this heparin solution (contained 97 mg Hep) was mixed with the aforementioned spongy chitosan and this was allowed to stand for about 24 hours at room temperature. This was followed by removal of the mixture and washing with deionized water and then immersion of the gel in a large amount of deionized water and stirring the deionized water with a magnetic stirrer (600 rpm) to remove the excess heparin and inorganic phosphate salts. This processes were repeated until the conductivity of the deionized water had declined sufficiently and become stable. The final conductivity was 100 µS/cm. After washing, the mixture was lyophilized to obtain a spongy dry material of a complex of chitosan and heparin (Hep/CT). Its weight was approximately 250 mg.

A spongy dry material of a complex of chitosan and chondroitin sulfate E (CS-E/CT) was similarly obtained using chondroitin sulfate E in place of the heparin. Its weight was approximately 240 mg.

Each of these dry materials was cut to approximately 1 cm² and used in the following Example 4.

### < Example 4 > Pharmacological testing

### (1) Preparation of the full-thickness skin wound model

Removal of the hair on dorsal side was carried out on 7-to-10-week old male Wistar (Crlj : WI) rats or Sprague-Dawley (SD) rats and a full-thickness wound, in which all the layers of the skin (epidermal layer, dermal layer, subcutaneous tissue) were removed, was prepared by excision with a surgical blade of an area of approximately 1 cm². This full-thickness wound model is a common and widely used model for evaluating tissue repair. The test substance was put on the full-thickness wound site (referred to below as the "affected part") and was fixed with a film dressing (Tegaderm) and an elastic bandage.

For the Control, the test substance was not applied and treatment was carried out with only the film dressing and elastic bandage.

### (2) Evaluation of hemorrhaging

The hemorrhaging due to the particular test substance was evaluated on the day after application of the test substance to the affected part, by taking a photograph of the affected part and performing a visual inspection, wherein the observation of an overflowing hemorrhaging from the wound site was scored as "++", the observation of an oozing hemorrhaging was scored as "+", and the absence of hemorrhaging was scored as "-".

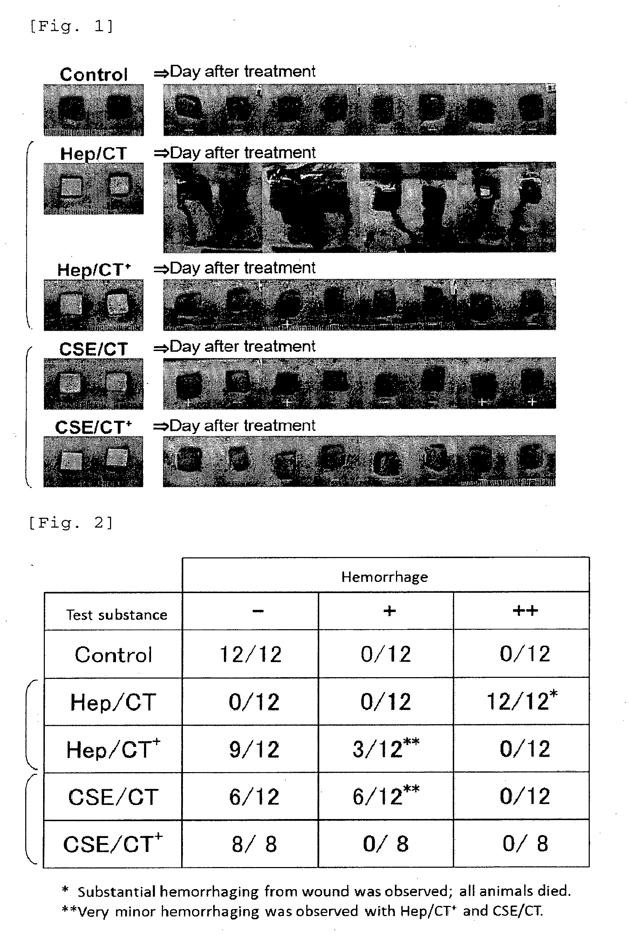

The results for the use of the Hep/CT and CS-E/CT produced in Reference Example 1 and the Hep/CT⁺ and CS-E/CT⁺ produced in Example 3 as the test substances are given in Fig. 1 (photograph) and Fig. 2.

The results for the application of Hep/CT (corresponds to the skin wound healing agent described in PTL 1) were "++" for hemorrhaging at 12 of the 12 wounds and the death of all of the animals by the day after application of the test substance.

In contrast to this, with the application of Hep/CT⁺, substantial hemorrhaging (++) was absent; "+" occurred at 3 wounds; "-" occurred at 9 wounds; and no animal mortality was observed.

In addition, when chondroitin sulfate E, another sulfated glycosaminoglycan, was used, hemorrhaging was observed at about half of the affected parts to which CS-E/CT was applied, i.e., among the 12 wounds, "++" occurred at 0 wounds, "+" occurred at 6 wounds, and "-" occurred at 6 wounds. However, for the affected parts to which CS-E/CT⁺ was applied, 8 of the 8 wounds were "-" and hemorrhaging was thus clearly inhibited.

These results demonstrated that the hemorrhagic activity caused by sulfated glycosaminoglycans could be remarkably inhibited by the use of a cationized chitosan. Moreover, an inhibitory effect on hemorrhaging was also seen for the complex comprising cationized chitosan and sulfated glycosaminoglycan, for the wound dressing containing this complex, and for the drug for treating a biological tissue that had this complex as an active ingredient. Because animal mortality was not seen with the use of this cationized chitosan and these complexes, wound dressings, and drugs for treating biological tissue, safe use on biological tissue was also confirmed.

### (3) Evaluation of the repair-promoting effect for biological tissue

The degree of epidermal extension (epidermal area) was measured on the seventh day after application of the test substance to the affected part. The epidermal extension is commonly used for the evaluation of full-thickness skin wounds and is an evaluation index that reflects the degree of tissue repair. The epidermal area was measured by tracing, onto a transparent sheet, the portion of the epidermis that extended towards the center from the edge of the full-thickness wound that had been made, and converting the area into a numerical value using image analysis software.

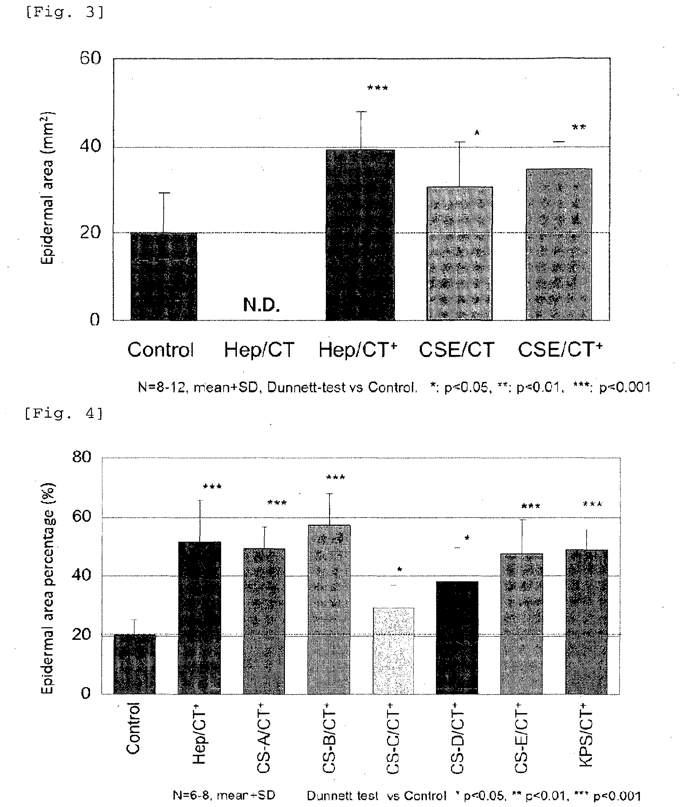

The evaluation was first carried out using the Hep/CT and CS-E/CT produced in the test example and the Hep/CT⁺ and CS-E/CT⁺ produced in Example 3. The results are shown in Fig. 3.

For the animals that received Hep/CT (corresponds to the skin wound healing agent described in PTL 1), the epidermal area could not be measured due to the death of all the animals at the day after application of the test substance as noted in (2) above.

A significant increase in the epidermal area was recognized for both Hep/CT⁺ at 39.3 ± 8.6 (SD) mm² and CS-E/CT⁺ at 34.9 ± 6.2 (SD) mm² versus the epidermal area for the Control of 20 ± 9.4 (SD) mm². With regard to the CS-E/CT, while hemorrhaging was observed as noted above, a significant increase in the epidermal area at 30.9 ± 10.1 (SD) mm² was ovserved with regard to tissue repair.

These results demonstrated that, even when a cationized chitosan is used, the effect of promoting biological tissue repair (wound healing) that is exhibited by sulfated glycosaminoglycans is in no way impaired. It was thus shown that the complex comprising cationized chitosan and sulfated glycosaminoglycan, the wound dressing containing this complex, and the drug for treating a biological tissue that has this complex as an active ingredient, exhibit an excellent promotion of biological tissue repair (wound healing) while also inhibiting hemorrhaging.

The biological tissue repair effect was also similarly evaluated for the cationized chitosan and sulfated glycosaminoglycan complexes prepared in Example 3 (Hep/CT⁺, CS-A/CT⁺, CS-B/CT⁺, CS-C/CT⁺, CS-D/CT⁺, CS-E/CT⁺, KPS/CT⁺). The epidermal area percentage was determined by calculating the percentage (%) for the epidermal area with reference to the area of the entire full-thickness wound. The results are given in Fig. 4.

Relative to an epidermal area percentage for the Control of 20.0 ± 5.3 (SD)%, a remarkable increase in the epidermal area percentage was confirmed in all instances for the Hep/CT⁺ at 51.5 ± 14.3 (SD)%, the CS-A/CT⁺ at 49.4 ± 7.3 (SD)%, the CS-B/CT⁺ at 57.1 ± 10.8 (SD)%, the CS-C/CT⁺ at 29.2 ± 7.7 (SD)%, the CS-D/CT+ at 38.1 ± 11.6 (SD)%, the CS-E/CT⁺ at 47.6 ± 11.4 (SD)%, and the KPS/CT⁺ at 48.8 ± 7.0 (SD)%.

Neither hemorrhaging from the affected part nor animal death was observed in these tests.

These results again demonstrated that, even when a cationized chitosan is used, the effect of promoting biological tissue repair (wound healing) that is exhibited by sulfated glycosaminoglycans is in no way impaired. It was thus shown that the complex comprising cationized chitosan and sulfated glycosaminoglycan, the wound dressing containing this complex, and the drug for treating a biological tissue that has this complex as an active ingredient, exhibit an excellent promotion of biological tissue repair (wound healing) while also inhibiting hemorrhaging.

### < Reference Example 2 > Production of cationized chitosan sponge

### (1) Production of cationized chitosan

A cationized chitosan was produced according to the method described in PTL 3. Specifically, 105 mL of 80 v/v% isopropyl alcohol was added to 7.5 g of microparticulated chitosan produced by the same method as in Example 1 and stirring was carried out at 60°C. This was followed by the addition of 10.5 mL of glycidyltrimethylammonium chloride (Aldrich) and reaction at 60°C for 7 hours under stirring condition. After the completion of the reaction, the reaction product was separated from the reaction solvent using a glass filter. The separated reaction product was washed with 20v/w ethanol and acetone. The reaction product was subsequently dried at 40°C under reduced pressure to obtain 12.5 g of a cationized chitosan (N-(2-hydroxypropyl)-3-trimethylammonium chitosan chloride, a quaternary chitosan).

### (2) Production of cationized chitosan sponge

250 mg of the cationized chitosan prepared in (1) above was dissolved homogeneously in 8 mL of water for injection, and the resulting solution was lyophilized to obtain a spongy dry material. This dry material was white and had a weight of approximately 240 mg. This dry material was cut as appropriate size and used in the following Reference Example 3.

### < Reference Example 3 > Pharmacological testing

### (1) Preparation of a burn wound model

The abdominal side of 10-week old male Sprague-Dawley (SD) rats was removed followed by treatment for 3 seconds with boiling water at 100°C over an area of approximately 7 cm². The necrotic tissue was removed from the treated area 3 days after the boiling water treatment to provide a skin wound. This burn wound model is a common and widely used model for evaluating tissue repair. The test substance was put on this burn wound area and was fixed with a film dressing (Tegaderm) and an elastic bandage. In the Control, treatment was carried out without the application of the test substance using only the film dressing and elastic bandage.

The degree of epidermal extension (epidermal area, epidermal area percentage) was measured on the tenth day after application of the test substance. Epidermal extension is commonly used for the evaluation of full-thickness skin wounds and is an evaluation index that reflects the degree of tissue repair. The epidermal area was measured by tracing, onto a transparent sheet, the area of the epidermis that extended towards the center from the edge of the wound that had been made, and converting the area into a numerical value using image analysis software. The epidermal area percentage was calculated as the percentage (%) for the area of the epidermis-covered portion with reference to the total wound area.

### (2) Evaluation of the biological tissue repair effect for burn wounds

An evaluation was carried out as to whether CT⁺ exhibited a wound-healing effect versus the Control.

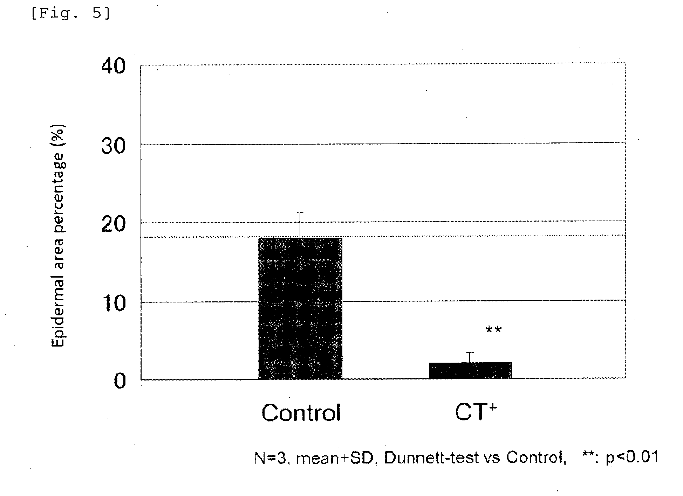

A significant reduction in the epidermal area percentage was observed for the cationized chitosan (CT⁺) by itself at 2.0 ± 1.3 (SD)% versus the Control at 17.9 ± 3.2 (SD)% (Fig. 5) .

These results demonstrated that CT⁺ does not by itself exhibit a wound-healing effect, but rather causes a delay in healing. Sulfated glycosaminoglycan cannot be adapted for wound healing due to the hemorrhage risk, while CT⁺ cannot be adapted to wound healing due to a retardation of wound healing. It has been shown with the present invention that the combination of the two inhibits the hemorrhage risk while also exhibiting a promoting effect on wound healing.

### Industrial Applicability

The present invention can be applied to drugs, medicines, and so forth.

## Claims

1. A complex comprising N-(2-hydroxypropyl)-3-trimethylammonium chitosan chloride and chondroitin sulfate A, chondroitin sulfate B or chondroitin sulfate E.

2. A wound dressing that comprises the complex according to claim 1.

3. A drug for use in healing wound, the drug comprising the complex according to claim 1 as an active ingredient.

## Patentansprüche

1. Komplex, umfassend N-(2-Hydroxypropyl)-3-trimethylammoniumchitosanchlorid und Chondroitinsulfat A, Chondroitinsulfat B oder Chondroitinsulfat E.

2. Wundverband, der den Komplex nach Anspruch 1 umfasst.

3. Arzneimittel zur Verwendung bei der Wundheilung, wobei das Arzneimittel den Komplex nach Anspruch 1 als aktiven Bestandteil umfasst.

## Revendications

1. Un complexe comprenant du N-(2-hydroxypropyl)-3-triméthylammonium chlorure de chitosan et du sulfate de chondroïtine A, du sulfate de chondroïtine B ou du sulfate de chondroïtine E.

2. Un pansement qui comprend le complexe selon la revendication 1.

3. Un médicament pour utilisation pour la cicatrisation, le médicament comprenant le complexe selon la revendication 1 en tant que principe actif.
